# EUROPEAN PATENT SPECIFICATION

(11) **EP 1 357 875 B1**
(45) Date of publication and mention of the grant of the patent: **13.04.2005**
(21) Application number: 01979174.8
(22) Date of filing: 26.10.2001
(51) Int. Cl.: A61F 13/472, A61L 15/60

(54) **ABSORBENT ARTICLE WITH ANATOMICALLY FORMED RAISED PORTION OF A DEHYDRATED HYDROGEL**
SAUGFÄHIGER ARTIKEL MIT EINEM ANATOMISCH GEFORMTEN ERHABENEN ABSCHNITT AUS DEHYDRATISIERTEM HYDROGEL
ARTICLE ABSORBANT AYANT UNE SECTION EN GEL DESHYDRATE DE FORME ANATOMIQUE EN RELIEF

(30) Priority: 24.11.2000 SE 0004324
(43) Date of publication of application: 05.11.2003
(73) Proprietor: SCA Hygiene Products AB, 405 03 Göteborg (SE)
(72) Inventor: KÖLBY-FALK, Ewa, S-416 78 Göteborg (SE)
(74) Representative: Romare, Laila Anette
(86) International application number: PCT/SE2001/002348
(87) International publication number: WO 2002/041817

(56) References cited:
- EP-A1- 0 834 296
- EP-A2- 1 099 447
- WO-A1-00/02509
- WO-A1-99/48451
- GB-A- 2 319 186
- US-A- 5 731 365
- US-A- 5 968 026

## Description

### TECHNICAL FIELD:

The invention relates to an absorbent product intended for female users, in which the product has a substantially elongated form, having a longitudinal direction, a transverse direction and a thickness direction, a liquid-permeable top side, a liquid-impermeable bottom side, two side edges extending in the longitudinal direction, and comprises an absorption body arranged between the top side and the bottom side.

### BACKGROUND:

Absorbent products such as sanitary towels and panty liners are intended to be worn in close contact with the body of the user. An absorbent product of this kind is usually fitted inside the briefs of the user and is held in contact with the body, during use, by the pressure from the briefs.

A common problem associated with absorbent products of this type is that the product has to be relatively narrow to be accommodated in the briefs, which means that there is a high risk of body fluid leaking out past the longitudinal side edges of the product. Such leakage is obviously extremely undesirable, since there is a consequent risk of the clothes of the user becoming soiled. Owing to the fact that the product also has a fairly small extent in the longitudinal direction, nor is it uncommon for fluid to leak out frontwards and rearwards past the end edges of the product. A specific problem here is rearward leakage, which usually occurs in connection with the user lying down, for example at night.

The most common cause of edge leakage is that the absorbent product is deformed during use, when the product is compressed between the thighs of the user. This causes folds to be formed, in an uncontrolled manner, in both the absorption core of the product and in its casing material. Such folds result in channels being formed on the surface of the product, in which body fluid can run out past the side edges. The compression of the product further leads to a reduction in the area available for the reception of fluid, so that there is a considerable risk of body fluid ending up alongside the product.

It has further become increasingly common for women to wear a thong, as it is known, i.e. briefs having an extremely small rear section. One problem with this is that it is virtually impossible to fasten a conventionally shaped sanitary towel, or a panty liner, in such a way that the towel or panty liner sits correctly in relation to the body of the user and, in addition, is held in place throughout its use. Another major problem is associated with the fact that thongs are often used for aesthetic reasons, since they are almost invisible even under dingy clothes and do not give rise to unsightly edge lines or creases on the clothes. To solve this problem, it has been proposed to design an absorbent product which is adapted to the shape of a thong. Such products are described in WO 97/39713 and in SE 9803981-1. The adaptation of the shape of the absorbent product means, however, that the product has to be designed with an extremely narrow rear section. One difficulty in this context is to produce a product having sufficiently high absorption capacity. It is additionally necessary for the product to be able to be placed in such a way in relation to the body of the user that no fluid runs out past the edges of the product and that the absorption capacity of the product can be fully utilized.

Certain types of absorbent products, such as sanitary towels and incontinence pads for mildly incontinent female users, are intended to be worn in close contact with the body of the user and, during use, to be in contact with the mucous membranes of the user in the genital area.

It is important in this context that the surface of an absorbent product which is intended to be facing the body of the user should be soft and comfortable and should not cause irritation. Moreover, the surface of the product must have the capacity to take up the body fluid which is delivered to the product and must rapidly allow the fluid to pass into the product and be absorbed by it. If the fluid is not admitted fast enough into the product, there is a clear risk of the fluid instead running onto the surface of the product and causing leakage. Moreover, the surface of the product becomes wet and sticky, something which is felt to be extremely uncomfortable by the majority of users. A wet surface can further cause the user discomfort in the form of skin irritation.

In order to avoid wet surfaces on absorbent products, these are generally provided with a liquid-permeable surface layer of a comparably hydrophobic material. Examples of such hydrophobic materials are perforated plastics films, plastics gauzes and non-woven materials made from hydrophobic fibres. By non-woven materials are meant various types of non-woven, bonded fibre layers. Hydrophobic layers of this kind have very low wettability and are therefore usually treated with tensides, for example, to increase the wettability and the capacity to let through liquid. The hydrophobic surface layers have a very dry surface, even after wetting, and are therefore comfortable to wear in contact with skin. One problem, however, is that when the relatively very dry surface of a conventional hydrophobic surface layer comes into contact with mucous membranes in the region of the genitalia of the user, this can cause discomfort in the form of chafing or irritation of the mucous membranes. To solve this problem, in WO 98/33463 it has been proposed to provide an absorbent product with a hydrophilic absorbent surface material within the region of the product which can enter into contact with the mucous membranes of the user.

One way of reducing the risk of edge leakage caused by deformation of the product during use is to provide the product with a preformed elevation, which, during use, is intended to bear against the genitalia of the user. Secreted body fluid can thereby be captured as soon as it leaves the body of the user and is immediately absorbed into the product instead of running out over its surface.

Previously known absorbent products provided with a fluid-receiving elevation do, however, suffer from a number of disadvantages.

A common way of creating an elevation has been to build it up quite simply by arranging a larger quantity of absorption material within the region of the elevation. Since the absorption material which is most often used is cellulose fluff material, as it is known, such an elevation collapses, however, and loses its shape when wetted. In order to produce an elevation which is sufficiently large even in wet state, an elevation consisting of cellulose fluff material has to contain so much absorption material that it is altogether too high, hard and uncomfortable to wear in the dry state.

In EP 0 335 252 and EP 0 335 253, it has been proposed to provide an absorbent product with a deformation element. The deformation element is acted upon by the transverse compression forces between the thighs of a user. The object of the deformation element is, during use, to bulge a section of the product in the direction of the body of the user. It is impossible, however, to fully control or predict the shape which the product will assume for each individual user. Nor is it possible to ensure the contact between the body of the user and the surface of the product, since the degree of bulging is wholly determined by the extent to which the product is compressed in the transverse direction.

A further problem associated with absorbent products in which the anatomical fit is critical is to keep the shield in place during use. If the product is conventionally fixed in the briefs of the user by glue or the like, it will move together with the briefs whenever the user moves. This means that it is at risk of moving out of position and losing contact with the body of the user. When absorbent products intended for wearing inside a pair of normal briefs have to be made relatively small in order to be accommodated in the briefs, even minor displacements of the product result in the risk of leakage past the product being drastically increased. This problem is especially manifest for products intended for use together with thongs. If the product is not fixed at all in the usage position, however, then it is at risk of falling out of the briefs or moving if a gap appears between the body of the user and the underclothes. Also, when visiting the toilet, it is disadvantageous if the product comes loose and, for example, falls down into the toilet.

There thus remains a need for an effective absorbent product which is comfortable to wear, sits securely in place during use and which also offers satisfactory leak-proofness and absorption capacity.

A product constructed according to the invention is primarily characterized in that the absorption body comprises an absorption part formed by a preformed, dehydrated hydrogel, which, as a result of the preforming, comprises a section which, when wetted, swells in the thickness direction and forms an elevation (6) on the top side of the product

Through pre-forming of the elevation, it is possible to achieve an especially good anatomical fit, which helps to produce good securement of the product during use, a high level of comfort and high leak-proofness. After swelling, the absorbent hydrogel acquires a slightly sticky surface, which adheres to the body of the user and serves further to ensure the securement of the product. A suction force is also generated in the absorption material during the course of the absorption, which helps to ensure that the shield is secured against the body of the user.

The hydrogel is made from a material which does not irritate the mucous membranes of the user and can comprise polymers based on vinyl alcohol, polyacrylate, polymethacrylate, polymethylene oxide, polysaccharide, acrylamide, vinyl pyrrolidone, or urethane, and mixtures, copolymers and derivatives thereof. It is also possible to use protein-based hydrogels.

The elevation of the absorbent product is given an anatomically adapted shape of predetermined height, width and length, expediently through casting of swollen hydrogel. The formed, swollen hydrogel is dehydrated and thereby essentially loses its three-dimensional shape, which is a great advantage when the absorbent product has to be packed and stored prior to use. When the shield is used, the hydrogel will absorb body fluid and, in doing so, will swell to its original, predetermined shape. At the same time, the fluid absorption, as previously discussed, means that the hydrogel acquires a sticky surface, which, in a kind and gentle and comfortable manner, produces a slight adhesion of the shield to the mucous membranes between the labia of the user. The sticky surface remains moist during use, which results in the elimination of the risk that the mucous membranes of the user will dry out. Where conventional fibrous absorption materials and conventional surface materials, designed to conduct fluid rapidly away from the surface of the absorbent product, are used, drying- out of the mucous membranes is an evident problem. A dry surface on an absorbent product which is in contact with sensitive body surfaces, such as mucous membranes, entails, moreover, an increased risk of chafing and other mechanical irritation.

The adhesiveness of the wet hydrogel, together with the anatomically adapted shape of the elevation, means that the absorbent product sits securely in place during use, without chafing or otherwise irritating the mucous membranes of the user. The extremely good fit and secure fastening make it possible to design the product to be small and discreet, without thereby jeopardizing leak-proofness. This means that the product can be superbly well designed to fit in a thong.

Where greater absorption capacity is required or the expected fluid flow is relatively large, the fluid-receiving capacity of the absorbent product can be increased by arranging an absorption material of higher fluid-reception rate inside the elevation of the shield. One suitable such absorption material is absorbent foam. The inner absorption material is expediently exposed to the reception of fluid through an opening in the hydrogel on the top side of the absorbent product.

According to an alternative embodiment, the absorbent product can be designed with an elevation in which there are arranged one or more cavities, or wells, which are open for the inflow of fluid on the top side of the product. Fluid which cannot immediately be absorbed by the hydrogel can temporarily be captured in the wells and can subsequently be gradually absorbed by the hydrogel. Such an embodiment is a simpler variant of the previously disclosed embodiment having an inner absorption body in the elevation.

The fact that the absorbent product according to the invention has an elevation made from a material with great capacity to absorb and chemically bond liquid in a hydrogel makes the product eminently suitable for use together with thongs. As previously discussed, absorbent products which are to fit in thongs have a very narrow rear section, whereby the available fluid-receiving surface of the product is smaller than for conventional absorbent products. When it swells during absorption, an elevation according to the invention can absorb large quantities of body fluid in relation to its original volume. It is usually calculated that a panty liner must have an absorption capacity of ca. 3-5 ml and a sanitary towel must be able to absorb ca. 12-15 ml. For products intended, for example, for night use or for incontinence protection, still greater absorption capacity can be necessary.

The height of the elevation above the surface of the absorbent product should be adjusted so that good contact between the body of the user and the elevation is ensured during use. The elevation should not, however, be so high that, during use, it presses against the body of the user and thereby creates discomfort. It is further necessary to ensure that the elevation does not chafe the sensitive soft parts in the crutch of the user. An elevation which, in its highest section, protrudes at least 5 mm from the surface of the product, but no more than 20 mm, has been shown to meet the dual requirements of good body contact and high user comfort.

An elevation according to the invention is relatively narrow, expediently max. 25 mm at the base, and preferably between 12 mm and 16 mm. At the top, the elevation is expediently between 3 mm and 10 mm wide, and preferably between 4 mm and 6 mm. The elevation is formed with a substantially triangular cross section and it is therefore wider at the base than at the top. The relatively narrow configuration of the elevation enables it to protrude somewhat between the labia of the user without causing her discomfort. It is here an advantage if the elevation parts a little on the labia, since the transfer of fluid from the user to the product is thereby facilitated.

It is important that the body fluid which is secreted should immediately be captured and absorbed into the elevation. This prevents any collection of fluid between the body of the user and the elevation. When the user stands up or otherwise moves, there is namely a risk of a gap appearing at the front part of the elevation, between the elevation and the body of the user. If a considerable quantity of unabsorbed fluid has been shut in between the body of the user and the surface of the product, such fluid can flow out through the gap. Sudden fluid flows of this kind are extremely undesirable, firstly because the user finds them uncomfortable, and secondly because they substantially increase the risk of leakage.

In order to connect to the anatomy of the user, the elevation should be highest at that part of the product which is intended to bear against the vaginal opening of the user. From the highest part, the height should gradually reduce in the direction of the end sections of the product. The elevation should extend between 5 mm and 40 mm, and preferably between 10 mm and 35 mm, rearwards from the highest section. In front of the highest section, the elevation should have a length of between 50 mm and 85 mm, preferably between 55 mm and 80 mm.

It is also expedient for the product to have an anatomically matched, planar form. Advantageously, the side edges of the product are therefore curved. The shaping of the product with a heavily tapered front section produces almost transverse edges, which, during use, hook onto the leg of the user and prevent the product from sliding rearwards. In order to obtain good hooking effect, it has been shown to be expedient for the front section to taper from its full width to a width corresponding to the smallest available width in the crutch region of the user within 5-20 mm of the length of the product. For anatomical adaptation to the space in the crutch region of the user and in order to prevent uncontrolled and undesirable deformation of the product, it has proved advantageous if the minimum width of the product is no greater than approx. 5 cm.

Where the product according to the invention is a sanitary towel, it expediently has a length not exceeding 260 mm. If extreme demands are placed upon discretion, the product can be designed with a total length of max. 155 mm.

The product can be provided with a fastening member for fastening the product in a pair of briefs. Expedient fastening members are, for example, adhesive, velcro fastening surfaces, friction linings, or the like. The fastening member can further comprise side flaps or wings, which expediently are arranged on the rear section of the product. Such side flaps are folded around the crutch of the briefs and fastened to the briefs, or one against the other.

### BRIEF DESCRIPTION OF THE FIGURES

The invention will be described in greater detail below with reference to the illustrative embodiments shown in the appended drawings, in which:
- Figure 1: shows a sanitary towel having an elevation according to the invention, viewed from the top side;
- Figure 2: shows a section along the line II-II through the sanitary towel in Figure 1;
- Figure 3: shows a sanitary towel for use in a thong and having an elevation according to the invention, viewed from the top side;
- Figure 4: shows a section along the line IV-IV through the sanitary towel in Figure 3;
- Figure 5: shows a sanitary towel having an elevation with a central well, viewed from the top side of the sanitary towel, and
- Figure 6: shows a section along the line VI-VI through the sanitary towel in Figure 5.

### DESCRIPTION OF EMBODIMENTS:

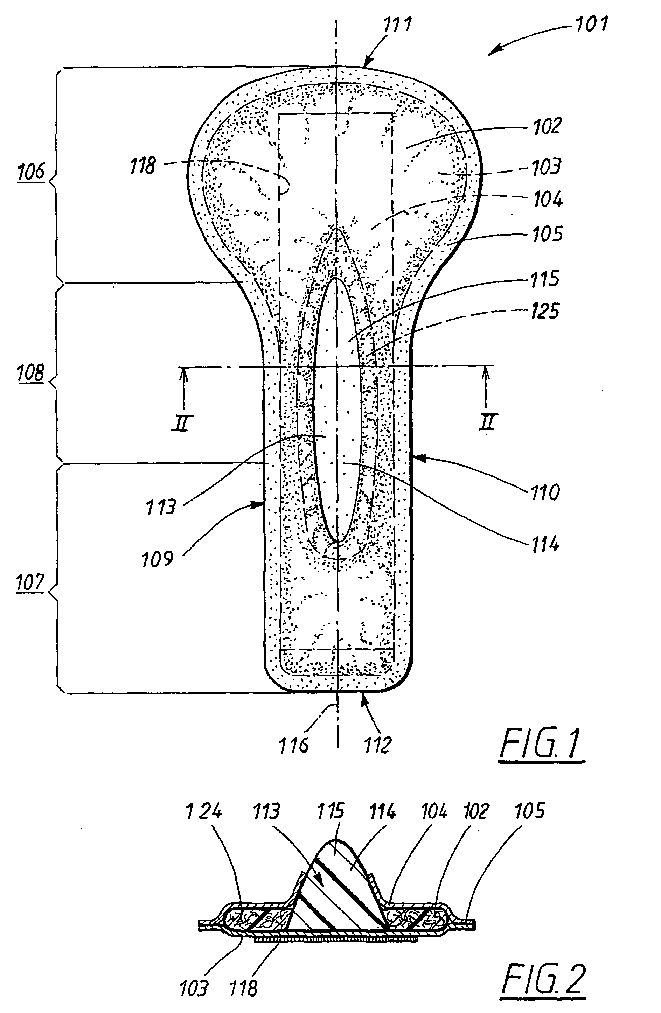

The sanitary towel 101 shown in Figures 1 and 2 comprises a liquid-permeable casing layer 102 arranged on the top side of the sanitary towel, i.e. that side of the sanitary towel which is intended to be facing a user when in use, a liquid-impermeable casing layer 103 arranged on the bottom side of the sanitary towel, i.e. that side of the sanitary towel which is intended to be facing away from the user, and an absorption body 104 arranged between the two casing layers. The two casing layers 102, 103 have essentially the same planar form as the absorption body 104, but have a somewhat larger extent in the plane, whereby they form a protruding edge 105 around the periphery of the whole of the absorption body 104. The casing layers 102, 103 are mutually connected within the protruding edge 105, for example by gluing, sewing or welding with heat or ultrasound.

The sanitary towel 1 is formed with a front section 106, a rear section 107 and an intermediate crutch section 108. The front section 106 has a rounded shape and is wider than the crutch section 108 and the rear section 109, which together form an essentially rectangular part situated behind the front section 106. In order to obtain good adaptation of the sanitary towel 101 to the space between the groins of the user and in order for the sanitary towel to be suitable for use together with a thong, it is expedient for the width of the crutch section 108 and the rear section 107 to be no greater than approximately 30-35 mm. From the comfort aspect, it is the width of the sanitary towel in the crutch section 108 which is here critical. It has been shown, in fact, that on all users there is a critical region between the groins, where the spacing between the muscles running down the inside of the thighs is approximately 30-35 mm. It is therefore expedient if the width of the crutch section 108 is no greater than approximately 30-35 mm, or at least is able to compress to this width without discomfort for the user. A sanitary towel or other absorbent product having a crutch section 108 which can easily be compressed can therefore be wider than 30-35 mm, but should not be wider than 60 mm in the crutch section 108 so as to prevent uncontrolled deformation which can make the product bulged and creased, and hence uncomfortable to wear, and which, moreover, can jeopardize leak-proofness. A relatively narrow crutch section 108 is also an advantage from the fastening aspect, since a sanitary towel in which the difference in width between the front section 106 and the crutch section 108 is large produces good hooking effect to the leg of the user and prevents the sanitary towel from sliding rearwards during use.

For the fit of the sanitary towel in a thong, the width of the rear section 107 is most significant, since it is desirable for the rear section 107 of the sanitary towel essentially to be able to accommodated between the edges of the rear section of the thong.

The sanitary towel 1 further has two longitudinal side edges 109, 110, a transverse front edge 111 and a transverse rear edge 112.

The liquid-permeable casing layer 102 expediently consists of a conventional, hydrophobic, liquid-permeable material. The liquid-permeable casing layer 102 is arranged over the surface of the absorption body 104 which is intended to be facing the user during use. Examples of suitable casing materials are perforated plastics films, hydrophobic non-woven materials, plastics gauzes, or the like. A hydrophobic surface material lets fluid through to the absorption body 104 within. Since the absorption body is more hydrophilic than the material in the liquid-permeable casing layer 102, the casing layer, after wetting, is almost fully drained of fluid. For this reason and since the casing layer 102, preferably, has essentially no absorption capacity, the layer 102 remains dry even after wetting.

When the sanitary towel 101 is used, it is placed in the region of the genitalia of the user, with the crutch section 108 of the sanitary towel situated next to the mouth of the urethra and the vaginal opening of the user. Secreted body fluid will consequently meet the sanitary towel 1 within a limited surface area thereof, the wet region as it is known. In the wet region, the liquid-permeable casing layer 102 has an opening 113 through which the absorption body 104 of the sanitary towel is exposed.

The liquid-permeable surface layer 103 is conventional in type and can therefore consist of any liquid-impermeable material whatsoever which is suitable for the purpose. Examples of such materials are various types of thin plastics films or non-woven materials which have been treated to withstand liquid penetration, for example by coating with plastic, wax or the like. Other treatments also, such as heat-calendering for melting an originally permeable material into a substantially liquid-impermeable layer, can be utilized. The liquid-impermeable surface layer 103 can further be constituted by a liquid-impermeable surface of the absorption body 104.

The absorption body 104 comprises a first part 114, which, after swelling, forms a longitudinal elevation 115 on the top side of the sanitary towel. The elevation 115 is centrally arranged along a centre line 116 extending in the longitudinal direction of the sanitary towel. The first part 114 of the absorption body 104 consists of a homogenous body of hydrogel having a cross section which, in the swollen state, is essentially triangular, as can be seen from Figure 2. In the course of the absorption and swelling, the elevation 115 consequently acquires an anatomically correct shape which tapers towards the top. An elevation 115 made from such material is soft in the wet state and has a certain resiliency and exhibits a moist, slightly sticky surface. The hydrogel further has a high absorption capacity. In the course of absorption, the material swells principally in the thickness direction and the elevation strives to regain the predetermined shape imparted to the hydrogel during manufacture of the sanitary towel 101. The fact that the gel is soft and flexible when wet means, however, that a certain forming also takes place according to the available space in the region of the genitalia of the user. An advantage with this is that the elevation 115 assumes a shape which is uniquely tailored to each user. In this way, both the leak-proofness and the comfort of the user are increased.

The central elevation 115 has an elongated shape and tapers in the direction of the end sections 106, 107 of the sanitary towel 101. The elevation 115 is highest in the region which, during use, is intended to be arranged next to the vaginal opening of the user. The length of the elevation 115 is expediently approximately 70 mm, but, if so desired, can range between 20 mm and 140 mm. From the highest section, the elevation expediently extends somewhat further rearwards than frontwards.

The absorption body 104 also comprises a second part 124, in the form of an essentially planar layer structure. In the example shown in Figures 1 and 2, the first part 114 of the absorption body 104 is arranged in a through hole 125 in the second part 124 and the elevation 115 thus, after swelling, protrudes through the hole 125 in the second part and through the hole 113 in the liquid-permeable casing layer 102. It is alternatively possible, of course, for the second part 124 of the absorption body 104 to be formed as a whole layer, without through holes, in which case the first part 114 of the absorption body is arranged on the second part 124. It is further possible to arrange the first part 114 in a recess in the second part 124.

The second part 124 of the absorption body 104 comprises one or more layer(s) of absorbent material, such as cellulose fluff material, absorbent bonded fibre layers, tissue layers, absorbent foam, peat, or the like. In addition, the body can contain non-absorbent components, such as stabilizing members, forming elements, bonding agents, etc. The absorption body 124 can further contain superabsorbent polymers, i.e. polymers with the capacity to absorb many times their own weight of liquid in forming a liquid-containing gel. Superabsorbents usually exist in the form of particles, flakes, fibres, granules or the like. The superabsorbent material can appear singly or together with another absorbent material. A suitable fibrous absorption material having high absorption capacity and good liquid-transporting capacity is the fibre material described in WO 94/10953 and WO 94/10956. These materials exist in the form of dry-formed fibre layers having high density and stiffness and are used directly in an absorbent product without first being defibrated.

The fact the elevation 115 consists of hydrogel which retains absorbed fluid means that there is no risk of the mucous membranes drying out, since the elevation 115, after wetting, will always have a moist surface. The mucous membranes are thereby kept moist during use and the risk of chafing and other irritation of the mucous membranes is virtually wholly eliminated.

During normal use of the sanitary towel 101, i.e. given moderate fluid flow and on the assumption that the sanitary towel 101 is correctly placed in relation to the body of the user, the liquid-permeable casing layer 102 will not primarily be wet by fluid. Instead, the secreted fluid first meets the first part 114 of the absorption body 104, which is exposed through the opening 113 in the liquid-permeable casing layer 102. Fluid which nevertheless lands beside the envisaged wet region passes in through the liquid-permeable casing layer 102 and is absorbed by the second part 124 of the absorption body 104. Also fluid which is primarily absorbed by the first part 114, once this has been saturated, is able to be transferred to the second part 124 of the absorption body 104. Owing to its low wettability, the liquid-permeable casing layer 102 acts as a barrier to the return passage of fluid from the sanitary towel 101. This means that even a sanitary towel which has absorbed a relatively large amount of fluid feels dry against the skin in the sections surrounding the wet region of the sanitary towel.

A fastening member 118 in the form of a rectangular region of self-adhesive glue extending in the longitudinal direction is arranged on the outside of the liquid-impermeable casing layer 103. When the sanitary towel 101 is used, this is fitted inside the briefs of the user and fixed in the briefs with the aid of the fastening member 118. Prior to use, the fastening member 118 is protected in a conventional manner, for example by covering with a protective layer of paper or plastic which has been silicone-treated or which has been stamped to allow easy detachment from the glue when the sanitary towel is about to be used. The glue can, of course, alternatively be arranged in any pattern whatsoever which is suitable for the purpose, such as a plurality of longitudinal strands, full coating, regions arranged only on the front section 106 and/or the rear section 107, or the like. Furthermore, other types of fastening members can be used, such as friction linings, press studs, clips, fastening tabs, or the like.

The fastening member 118 is not necessary to the invention but can be excluded. Since a product according to the invention has good anatomical fit and since the hydrogel in the absorbent elevation serves to adhere the elevation to the body of the user, a sanitary towel, or other absorbent product according to the invention, can be held in place without being fixed in the briefs. This is especially the case for relatively small absorbent products such as panty liners, and sanitary towels and panty liners intended for wearing together with thongs.

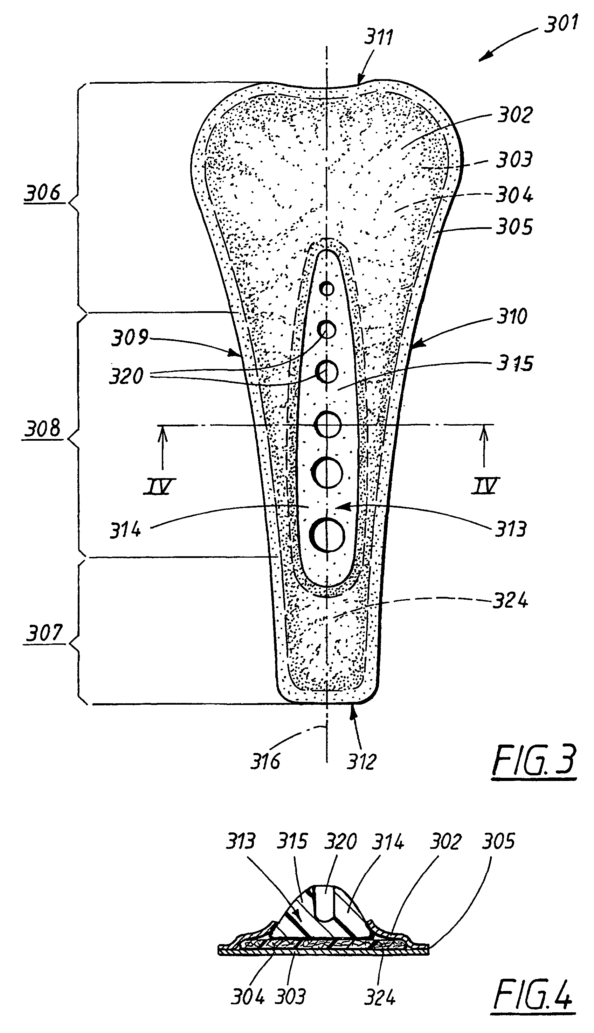

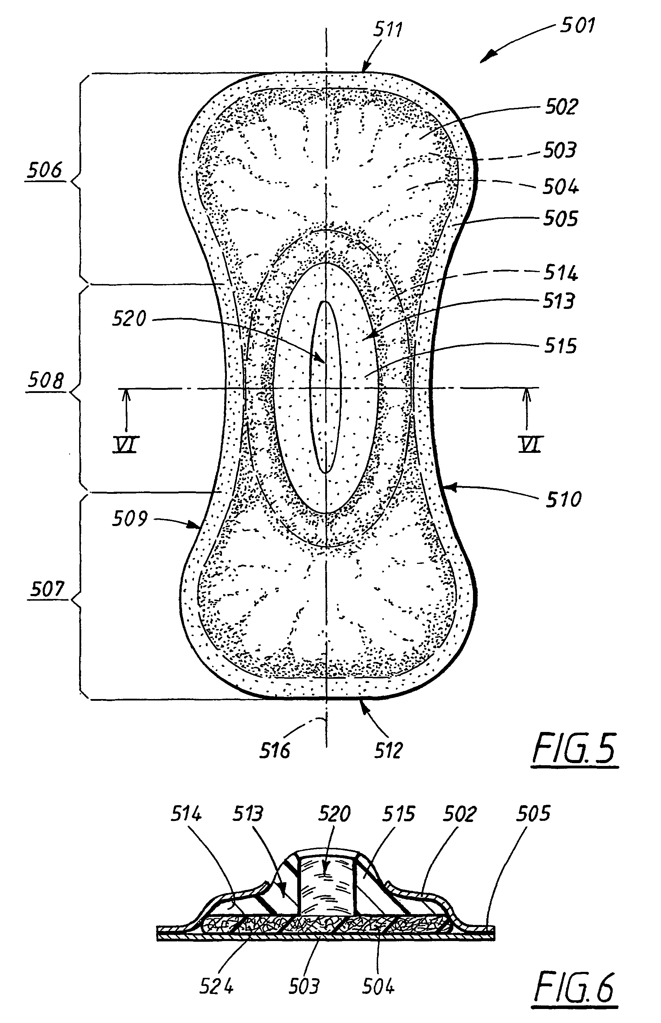

Like the sanitary towel 1 in Figure 1, the sanitary towel 301 shown in Figures 3 and 4 comprises a liquid-permeable casing layer 302, a liquid-impermeable casing layer 303 and an absorption body 304 arranged between the casing layers 302, 303 and consisting of two parts 314, 324, a first part of which, after swelling, forms an elevation 315 on the top side of the sanitary towel. The casing layers 302, 303 are mutually connected around the absorption body 304 and form a protruding casing edge 305.

The sanitary towel 301 has a substantially trapezoidal shape, having a wide front section 306 which tapers towards a rear section 307 via an intermediate crutch section 308. The sanitary towel 301 further has slightly inwardly curved side edges 309, 310, a front end edge 311 and a rear end edge 312. Such a shape is especially well suited to fitting in a thong.

The first part 314 of the absorption body 304 is arranged on top of the second part 324, at an opening 313 in the liquid-permeable casing layer 302 and, after swelling, will protrude through the opening 313 and form an elevation 315 on the top part of the sanitary towel. The elevation 315 extends centrally along the longitudinal centre line 316 of the sanitary towel 301, principally in the crutch section 308. Unlike the embodiment in Figures 1 and 2, the elevation 315 has a plurality of circular wells 320 arranged centrally one behind the other in the longitudinal direction of the elevation 315. The elevation 315 is preformed from hydrogel which has been dehydrated before having been placed on the second part 324 of the absorption body 304. Prior to the configuration of the elevation 315 during use, the first part 314 of the absorption body has an essentially planar form and the wells 320 are scarcely distinguishable to the naked eye. When the hydrogel in the first part 314 absorbs fluid and swells, the depth of the wells increases as the height of the elevation 315 increases, until the elevation has regained its swollen, predetermined shape and height. The wells 320 serve to collect fluid and prevent the fluid from running onto the surface of the elevation 315 before it has managed to be absorbed by the hydrogel. Fluid which has collected in the wells 320 can subsequently be absorbed by the hydrogel and can leave room for new fluid delivered to the sanitary towel.

The sanitary towel 501 shown in Figure 5 also comprises a liquid-permeable casing layer 502 arranged on the top side of the sanitary towel 501, a liquid-impermeable casing layer 503 arranged on the bottom side of the sanitary towel, and an absorption body 504 arranged between the two casing layers. The two casing layers 502, 503 have essentially the same planar form as the absorption body 504, but have a somewhat larger extent in the plane, whereby they form a protruding edge 505 around the periphery of the whole of the absorption body 504.

The sanitary towel 501 is hourglass-shaped, having wider end sections 506, 507 and an intermediate narrower crutch section 508, and has inwardly curved side edges 509, 510 and outwardly curved end edges 511, 512.

The absorption body 504 comprises a first part 514 made from hydrogel, which is arranged centrally along the longitudinal centre line 516 of the sanitary towel 501, on the second part 524 of the absorption body 504. The first part swells during use as it absorbs fluid and thereupon forms an annular elevation 515, having a centrally arranged, oval-shaped hole 520 extending the whole of the way through the first part 514, down to the second part 524 of the absorption body 504. The first part 514 of the absorption body 504 thus forms a fluid-receiving duct into the second part 514 of the absorption body. In the same way as in the wells 320 in the elevation 315 of the embodiment shown in Figures 3 and 4, the central opening 520 in the elevation 515 of the sanitary towel 501 in Figures 5 and 6 serves as a fluid-receiving member and as a reservoir for fluid which has not yet managed to be absorbed by the sanitary towel.

If so desired, the hole 520 in the elevation 515 can be wholly or partially filled with absorbent material, for example of the same type as in the second part 524 of the absorption body 504, or of some other material type. It is also possible to make a part of the second part 524 of the absorption body 504 protrude a bit through the hole 520 in the elevation 515. Another alternative is to fill the hole 520 wholly or partially with an essentially non-absorbent fibre wad, an open-celled foam material, or the like, which offers a roughly capillary structure which allows rapid inflow of fluid to the second part 524 of the absorption body 504.

The described sanitary towels are of the type which have a casing consisting of a liquid-permeable casing layer and a liquid-impermeable casing layer mutually connected in a connecting edge around an absorption body. This is not a design which is necessary to the invention, of course, but rather other occurring types of casing designs can be used, if so desired. It is possible, for example, to make the second part of the absorption body extend out between the casing layers and form part of the edge joint. It is further possible to use a single continuous outer casing layer, which is arranged as an envelope around an absorption body, and possibly a liquid-impermeable layer inside this. In the latter case, a liquid-impermeable surface can alternatively be arranged on the bottom side of the product through coating with plastic, wax or the like, or through the use of a plastics film which is perforated to produce liquid-permeability on the top side of the product.

The absorption bodies of the absorbent products can comprise further components apart from those described. Examples of such further components are bonding agents, further absorption layers, reinforcing layers, stiffening elements, elastic, etc.

Even though the sanitary towels 301, 501 shown in Figures 3-6 have been described without fastening members, it is also, of course, possible to provide such sanitary towels with fastening members in the same way as in the sanitary towel 1 shown in Figures 1-2.

The invention has been described above in connection with sanitary towels. However, it is also possible to utilize the invention for other absorbent products intended for wearing by female users. The invention also therefore embraces panty liners and incontinence pads for women.

## Claims

1. An absorbent product, in which the product has a substantially elongated form, having a longitudinal direction, a transverse direction and a thickness direction, a liquid-permeable top side, a liquid-impermeable bottom side, two side edges (109, 110) extending in the longitudinal direction, and comprises an absorption body (104) arranged between the top side and the bottom side, **characterized in that** the absorption body (104) comprises an absorption part (114) formed by a preformed, dehydrated hydrogel, which, as a result of the preforming, comprises a section which, when wetted, swells in the thickness direction and forms an elevation (115) on the top side of the product.

2. An absorbent product according to Claim 1, in which the elevation (115), after wetting, exhibits a sticky surface on the top side of the product.

3. An absorbent product according to Claim 1 or 2, in which the hydrogel comprises polymers based on vinyl alcohol, polyacrylate, polymethacrylate, polymethylene oxide, polysaccharide, acrylamide, vinyl pyrrolidone, or urethane, and mixtures, copolymers and derivatives thereof.

4. An absorbent product according to any one of the preceding claims, in which the elevation (315; 515) has an inner cavity (320; 520), or well, which is formed in the hydrogel and which has an opening on the top side of the product.

5. An absorbent product according to Claim 4, in which the inner cavity (320; 520) or the well in the elevation (315; 515) comprises a porous medium.

6. An absorbent product according to Claim 5, in which the porous medium comprises a fibrous material.

7. An absorbent product according to Claim 5 or 6, in which the porous medium is an absorbent material.

8. An absorbent product according to Claim 7, in which the absorbent material comprises an absorbent foam material.

9. An absorbent product according to any one of the preceding claims, in which the elevation (115), after swelling, has a maximum height of 5-20 mm.

10. An absorbent product according to any one of the preceding claims, in which the elevation (115) has a maximum width of 25 mm and preferably a maximum width of 12-16 mm.

11. An absorbent product according to any one of the preceding claims, in which the elevation (115) has a length of 30-100 mm.

12. An absorbent product according to any one of the preceding claims and having a front section (106), which is arranged to be facing frontwards on a user, a rear section (107), which is arranged to be facing rearwards on a user, and a crutch section (108), which is situated between the front section (106) and the rear section (107), the rear section (107) having a maximum width not exceeding 30 mm.

13. An absorbent product according to any one of the preceding claims and having a front section (106), which is arranged to be facing frontwards on a user, a rear section (107), which is arranged to be facing rearwards on a user, and a crutch section (108), which is situated between the front section (106) and the rear section (107), the crutch section (108) having a maximum width not exceeding 35 mm.

## Patentansprüche

1. Absorbierendes Produkt, wobei das Produkt eine im wesentlichen längliche Form mit einer Längsrichtung, einer Querrichtung und einer Stärkerichtung, eine flüssigkeitsdurchlässige Oberseite, eine flüssigkeitsundurchlässige Unterseite, zwei Seitenkanten (109, 110), die sich in der Längsrichtung erstrecken, aufweist, und einen Absorptionskörper (104), der zwischen der Oberseite und der Unterseite angeordnet ist, umfasst, **dadurch gekennzeichnet, dass** der Absorptionskörper (104) einen Absorptionsteil (114) umfasst, der durch ein vorgeformtes dehydriertes Hydrogel gebildet ist, das als eine Folge des Vorformens einen Abschnitt umfasst, der wenn er benetzt wird, in der Stärkerichtung quillt und eine Erhebung (115) auf der Oberseite des Produkts bildet.

2. Absorbierendes Produkt nach Anspruch 1, bei dem die Erhebung (15) nach dem Benetzen auf der Oberseite des Produkts eine klebrige Oberfläche aufweist.

3. Absorbierendes Produkt nach Anspruch 1 oder 2, bei dem das Hydrogel Polymere auf Vinylalkoholbasis, Polyacrylate, Polymethacrylate, Polymethylenoxid, Polysaccharide, Acrylamid, Vinylpyrrolidon, oder Urethan und Mischungen, Copolymere und Derivate davon, umfasst.

4. Absorbierendes Produkt nach einem der vorstehenden Ansprüche, bei dem die Erhebung (315; 515) einen Innenhohlraum (320; 520) bzw. einen Schacht aufweist, die bzw. der in dem Hydrogel ausgebildet ist und die bzw. der eine Öffnung auf der Oberseite des Produkts aufweist.

5. Absorbierendes Produkt nach Anspruch 4, bei dem der Innenhohlraum (20; 520) bzw. der Schacht in der Erhebung (315; 515) ein poröses Medium umfasst.

6. Absorbierendes Produkt nach Anspruch 5, bei dem das poröse Medium faserförmiges Material umfasst.

7. Absorbierendes Produkt nach Anspruch 5 oder 6, bei dem das poröse Medium ein absorbierendes Material ist.

8. Absorbierendes Produkt nach Anspruch 7, bei dem das absorbierende Material einen absorbierenden Schaumstoff umfasst.

9. Absorbierendes Produkt nach einem der vorstehenden Ansprüche, bei dem die Erhebung (115) nach dem Quellen eine maximale Höhe von 5 bis 20 mm aufweist.

10. Absorbierendes Produkt nach einem der vorstehenden Ansprüche, bei dem die Erhebung (115) eine maximale Breite von 25 mm und vorzugsweise eine maximale Breite von 12-16 mm aufweist.

11. Absorbierendes Produkt nach einem der vorstehenden Ansprüche, bei dem die Erhebung (115) eine Länge von 30-100 mm aufweist.

12. Absorbierendes Produkt nach einem der vorstehenden Ansprüche, mit einem Vorderabschnitt (106), der derart angeordnet ist, dass er an einem Verwender nach vorne weist, einem Hinterabschnitt (107), der derart angeordnet ist, dass er an einem Verwender nach hinten weist und einem Schrittabschnitt (108), der zwischen dem Vorderabschnitt (106) und dem Hinterabschnitt (107) angeordnet ist, wobei der Hinterabschnitt (107) eine maximale Breite aufweist, die 30 mm nicht überschreitet.

13. Absorbierendes Produkt nach einem der vorstehenden Ansprüche, mit einem Vorderabschnitt (106), der derart angeordnet ist, dass er an einem Verwender nach vorne weist, einem Hinterabschnitt (107), der derart angeordnet ist, dass er an einem Verwender nach hinten weist und einem Schrittabschnitt (108), der zwischen dem Vorderabschnitt (106) und dem Hinterabschnitt (107) angeordnet ist, wobei der Schrittabschnitt (108) eine maximale Breite aufweist, die 35 mm nicht überschreitet.

## Revendications

1. Article absorbant, dans lequel l'article a une forme sensiblement allongée, ayant une direction longitudinale, une direction transversale et une direction d'épaisseur, une face supérieure perméable aux liquides, une face inférieure imperméable aux liquides, deux bords latéraux (109, 110) s'étendant dans la direction longitudinale, et comprend un corps absorbant (104) placé entre la face supérieure et la face inférieure, **caractérisé en ce que** le corps absorbant (104) comprend une partie absorbante (114) formée par un hydrogel déshydraté et préformé qui, suite au préformage, comprend une section qui, lorsqu'elle est mouillée, enfle dans la direction de l'épaisseur et forme une protubérance (115) sur la face supérieure de l'article.

2. Article absorbant selon la revendication 1, dans lequel la protubérance (115), après mouillage, présente une surface collante sur la face supérieure de l'article.

3. Article absorbant selon la revendication 1 ou 2, dans lequel l'hydrogel comprend des polymères à base d'alcool vinylique, d'acrylamide ou de vinyle pyrrolidone, des polyacrylates, polyméthacrylates, polyoxyméthylènes, polysaccharides ou polyuréthanes, et leurs mélanges, copolymères et dérivés.

4. Article absorbant selon l'une quelconque des revendications précédentes, dans lequel la protubérance (315 ; 515) a une cavité intérieure (320 ; 520), ou puits, qui est formé(e) dans l'hydrogel et qui comporte une ouverture sur la face supérieure de l'article.

5. Article absorbant selon la revendication 4, dans lequel la cavité intérieure (320 ; 520) ou le puits de la protubérance (315 ; 515) comprend un milieu poreux.

6. Article absorbant selon la revendication 5, dans lequel le milieu poreux comprend un matériau fibreux.

7. Article absorbant selon la revendication 5 ou 6, dans lequel le milieu poreux est un matériau absorbant.

8. Article absorbant selon la revendication 7, dans lequel le matériau absorbant comprend un matériau mousse absorbant.

9. Article absorbant selon l'une quelconque des revendications précédentes, dans lequel la protubérance (115), après gonflement, a une hauteur maximale comprise entre 5 et 20 mm.

10. Article absorbant selon l'une quelconque des revendications précédentes, dans lequel la protubérance (115) a une largeur maximale de 25 mm et de préférence une largeur maximale comprise entre 12 et 16 mm.

11. Article absorbant selon l'une quelconque des revendications précédentes, dans lequel la protubérance (115) a une longueur comprise entre 30 et 100 mm.

12. Article absorbant selon l'une quelconque des revendications précédentes et ayant une section avant (106), qui est destinée à être tournée vers l'avant sur un utilisateur, une section arrière (107), qui est destinée à être tournée vers l'arrière sur un utilisateur, et une section d'entrejambe (108), qui est située entre la section avant (106) et la section arrière (107), la section arrière (107) ayant une largeur maximale ne dépassant pas 30 mm.

13. Article absorbant selon l'une quelconque des revendications précédentes et ayant une section avant (106), qui est destinée à être tournée vers l'avant sur un utilisateur, une section arrière (107), qui est destinée à être tournée vers l'arrière sur un utilisateur, et une section d'entrejambe (108), qui est située entre la section avant (106) et la section arrière (107), la section d'entrejambe (108) ayant une largeur maximale ne dépassant pas 35 mm.
